# EUROPÄISCHE PATENTSCHRIFT

(11) **EP 1 245 190 B1**
(45) Veröffentlichungstag und Bekanntmachung des Hinweises auf die Patenterteilung: **17.12.2003**
(21) Anmeldenummer: 02005755.0
(22) Anmeldetag: 13.03.2002
(51) Int. Cl.: A61B 6/10

(54) **Obertisch-Durchleuchtungsgerät mit schwenkarretierbarem Röntgenstrahler**
X ray device mounted above a table with a swivelling x ray source having a brake
Appareil de radiographie monté au dessus d'une table avec une source de rayons X pivotante avec un frein

(30) Priorität: 26.03.2001 DE 20105190 U
(43) Veröffentlichungstag der Anmeldung: 02.10.2002
(73) Patentinhaber: SIEMENS AKTIENGESELLSCHAFT, 80333 München (DE)
(72) Erfinder: Fadler, Franz, 91077 Hetzles (DE); Knappe, Peter, 96050 Bamberg (DE); Leidenberger, Stefan, 91090 Effeltrich (DE)

(56) Entgegenhaltungen:
- DE-A- 3 009 496
- DE-A- 19 625 411
- DE-C- 4 200 654

## Beschreibung

Die Erfindung bezieht sich auf ein Obertisch-Durchleuchtungsgerät mit um die horizontale Achse des Strahlerstativs drehbarem Röntgenstrahler mit einer Schwenkarretierung mit einem handbetätigbarem Entriegelungshebel.

Die Entriegelung der Strahlerdrehung an einem Obertisch-Durchleuchtungsgerät befindet sich beispielsweise hinter dem Röntgenstrahler direkt an der Stelle des Strahlerträgers, an der sich die Drehebene zwischen dem Strahlerträger und dem horizontalen Arm des Strahlerstativs befindet. In dieser Ebene liegt ja auch der Rasthebel der Schwenkarretierung, sodass sich an dieser Stelle sehr einfach dessen handbetätigbarer Entriegelungshebel anordnen lässt. Dabei liegt der Entriegelungshebel allerdings in etwa 2 Meter Höhe über dem Boden und noch dazu auf der der Bedienerseite des Gerätes abgelegenen Seite, sodass eine normal gewachsene Bedienungsperson nahezu unmöglich mit der Hand an den Entriegelungshebel heranreicht.

Darüber hinaus sind auch bereits elektromagnetische Verriegelungen für den drehbaren Strahlerträger bekannt geworden, bei denen eine bedienerfreundliche Fernentriegelung möglich ist. Diese Anordnungen sind jedoch sehr aufwändig, teuer und gegenüber handbetägigbaren Schwenkarretierungen störungsanfälliger.

Ein Gerät gemäß Oberbegriff des Anspruchs 1 ist bereits bekannt durch die DE 196 25 411 A1.

Der Erfindung liegt daher die Aufgabe zugrunde, ein Obertisch-Durchleuchtungsgerät der eingangs genannten Art so auszugestalten, dass eine einfachere und leichter zugängliche Entriegelung der Strahlerdrehung möglich ist.

Zur Lösung dieser Aufgabe ist erfindungsgemäß vorgesehen, dass der Entriegelungshebel in einen im wesentlichen L- oder C-förmigen Handgriff mit einem in einer vertikalen Ebene liegenden Griffschenkel zur Verschwenkung des Strahlerträgers integriert ist.

Durch die erfindungsgemäße Verlagerung des Entriegelungshebels liegt er zwar nach wie vor in etwa 2 Meter Höhe, aber benachbart der Vorderkante des Patientenlagerungstisches an der die Bedienungsperson steht, sodass sie ohne über den Tisch nach hinten greifen zu müssen, die Entriegelungshebel wesentlich besser betätigen kann.

Die Übertragungseinrichtung kann dabei in unterschiedlichster Weise, beispielsweise als Zahnstangen- oder Zahnradgetriebe, ausgebildet sein. Bevorzugt kann man als Übertragungseinrichtung zur Verbindung des Entriegelungshebels mit dem Rasthebel der Schwenkarretierung eine Bowdenzug verwenden, der sich sehr einfach und ohne Behinderung der übrigen Einbauteile des Strahlerträgers in dessen Inneren verlegen lässt.

Neben der Möglichkeit, einen einfachen, als L-förmiger Schwenkhebel ausgebildeten Entriegelungshebel vorzusehen, hat es sich in Ausgestaltung der Erfindung als besonders zweckmäßig erwiesen, dass der Entriegelungshebel in einem, im Wesentlichen C-förmigen Handgriff des Strahlerträgers integriert ist.

Statt den Entriegelungshebel einfach leicht zugänglich als gesonderten Hebel am C-förmigen Handgriff des Strahlerträgers anzubringen, kann dabei gemäß einem weiteren Merkmal der Erfindung vorgesehen sein, dass der Handgriff des Strahlerträgers um die Achse seiner Befestigungsschenkel schwenkbar gelagert, direkt auch als Entriegelungshebel ausgebildet ist. Um eine Stahlerdrehung zu veranlassen, wird zunächst der Handhebel ein Stück nach unten geschwenkt, wobei über den Bowdenzug der Rasthebel der Schwenkarretierung gelöst wird und anschließend kann durch Verschwenken des so etwas heruntergeklappten Handgriffs die Strahlerdrehung erfolgen.

Weitere Vorteile, Merkmale und Einzelheiten der Erfindung ergeben sich aus der nachfolgenden Beschreibung eines Ausführungsbeispiels sowie anhand der Zeichnung. Dabei zeigen:
- Fig. 1: Eine schematische perspektivische Ansicht eines erfindungsgemäßen Obertisch-Durchleuchtungsgeräts,
- Fig. 2: eine vergrößerte perspektivische Ansicht des geöffneten Strahlerträgers schräg von vorn,
- Fig. 3: eine teilweise geöffnete Ansicht des Strahlerträgers nach Fig. 2 schräg von hinten und
- Fig. 4: eine der Fig. 2 entsprechende perspektivische Vorderansicht eines geöffneten vereinfachten Strahlerträgers mit einem einfachen Entriegelungshebel.

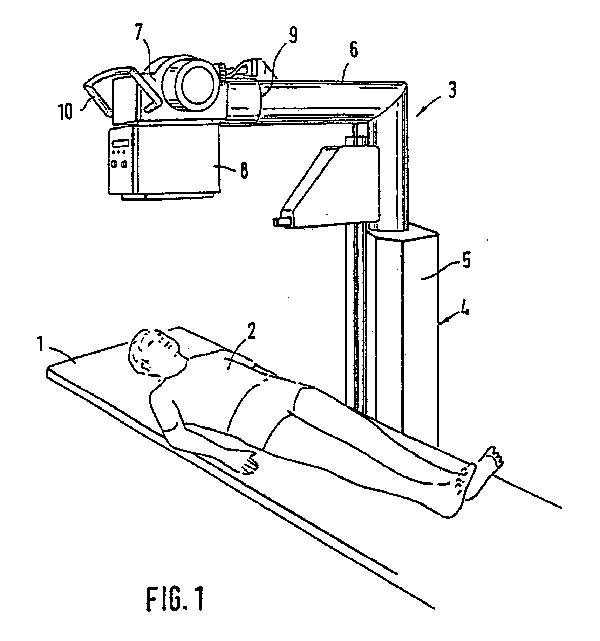

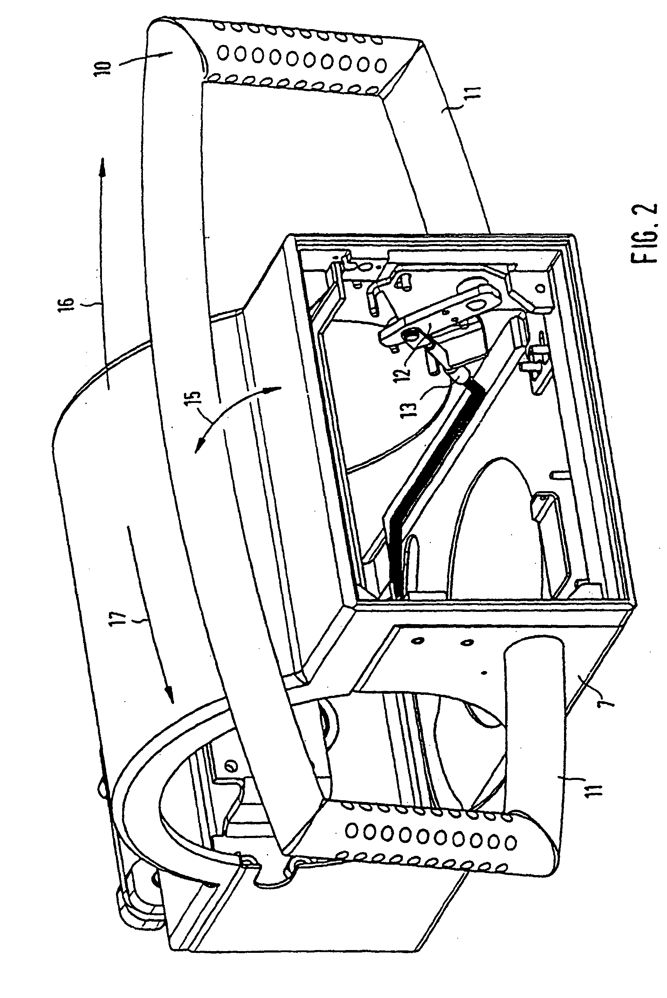

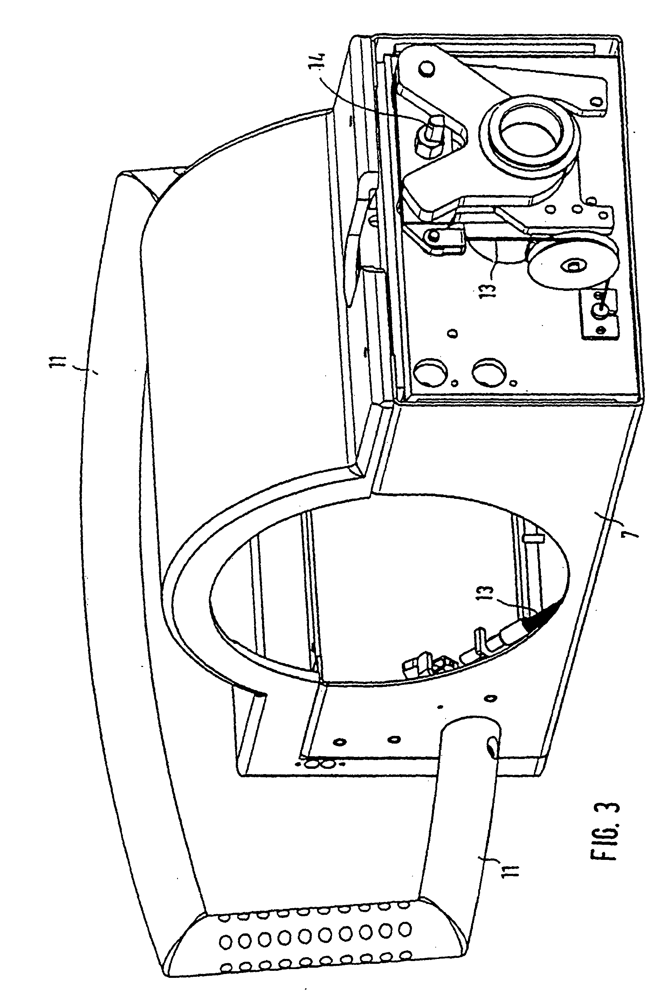

Die Fig. 1 zeigt schematisch einen, auf einem verstellbaren Patientenlagerungstisch 1 liegenden Patienten 2 unter einem Obertisch-Durchleuchtungsgerät 3 mit einem, im Wesentlichen L-förmigen Strahlerstativ 4 mit der tragenden Strahlersäule 5 und einem, im Wesentlichen horizontalen Trägerarm 6, an dem der Strahlerträger 7 für den eigentlichen Strahler 8 schwenkbar gelagert ist. Die Schwenkebene liegt dabei etwa in der Ebene 9 hinter dem Strahlerträger 7, sodass der bislang an dieser Stelle angeordnete Entriegelungshebel für die Schwenkarretierung für die vor dem Patiententisch 1 stehende Bedienungsperson, die also auf der der Säule 5 abgelegenen Seite des Strahlerstativs üblicherweise angeordnet ist, nur sehr schwer erreichbar war. Erfindungsgemäß ist deshalb bei der Anordnung nach den Figuren 1 bis 3 vorgesehen, dass der, im Wesentlichen C-förmige Handhebel 10, mit Hilfe dessen die Schwenkung des Strahlers 3 erfolgen soll, gleichzeitig auch als Entriegelungshebel ausgebildet ist, indem die beiden Befestigungsschenkel 11 des Handhebels 10 am Strahlergehäuse 7 schwenkbar gelagert sind. Auf dem einen der Befestigungsschenkel 11 ist am Ende ein Hebelarm 12 befestigt, der über einen Bowdenzug 13 mit einem Rasthebel 14 verbunden ist, der auf der Rückseite des Strahlerträgers 11 liegend in der Schwenkebene 9 in eine nicht gezeigte Verriegelungsschablone mit einer Mehrzahl von Einschnitten zur Einstellung unterschiedlicher Strahlerschwenkpositionen eingreifen kann. Zur Schwenkentriegelung wird der Handhebel 11 zunächst in Richtung des Doppelpfeils 15 nach unten geschwenkt, wodurch über den Hebel 12 und dem Bowdenzug 13 der Rasthebel 14 betätigt wird und aus seiner Arretierstellung herausbewegt wird. Anschließend kann durch Drehung des Handgelenks des Benutzers eine Verschwenkung des Handgriffs 10 in Richtung des Pfeils 16 oder des Pfeils 17 erfolgen und somit die gewünschte verdrehte Strahlerstellung eingestellt werden. Sobald die gewünschte Position gefunden ist, wird der Handhebel 10 in der Gegenrichtung der vorherigen Entriegelungsverschwenkung zurückbewegt und der Rasthebel 14 rastet in der nächstgelegenen Rastposition der Arretierschablone wieder ein. Dabei kann auch eine Feder im Bowdenzug vorgesehen sein, die beim Hochschwenken des Handhebels 10 den Rasthebel in die Arretierstellung vorspannt, sodass er in den nächsten Rasteinschnitt einrastet.

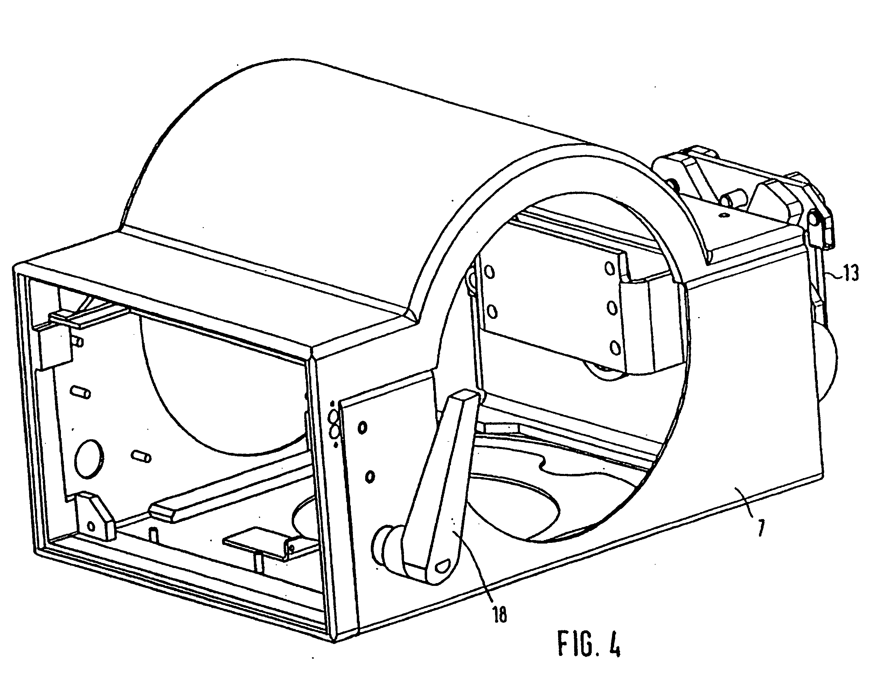

Die Fig. 4 zeigt eine vereinfachte Ausführungsform, bei der ein zum Verschwenken des Strahlerträgers 7 mit dem Strahler 8 dienender Handhebel 10 fehlt. Die Verschwenkung erfolgt hier durch direktes Ergreifen des Gehäuses des Strahlerträgers oder des Strahlers mit der einen und des als einfacher Hebel ausgebildeten Entriegelungshebel 18. Auf der Achse des Entriegelungshebels 18 sitzt wiederum ein mit einem Bowdenzug verbundener Übertragungshebel, entsprechend dem Hebel 12 beim Ausführungsbeispiel nach den Figuren 1 bis 3.

## Patentansprüche

1. Obertisch-Durchleuchtungsgerät mit um die horizontale Achse des Strahlerstativs drehbarem Röntgenstrahler mit einer Schwenkarretierung mit einem handbetätigbarem Entriegelungshebel der an der dem Strahlerstativ (4) abgelegenen Vorderseite des Strahlerträgers (7) angeordnet und über eine Übertragungseinrichtung mit dem Hebel (14) der Schwenkarretierung verbunden ist, **dadurch gekennzeichnet, dass** der Entriegelungshebel in einen, im Wesentlichen L- oder C-förmigen Handgriff (10) mit einem in einer vertikalen Ebene liegenden Griffschenkel zur Verschwenkung des Strahlerträgers (7) integriert ist.

2. Obertisch-Durchleuchtungsgerät nach Anspruch 1, **dadurch gekennzeichnet, dass** der Handgriff (10) des Strahlerträgers (7) um die Achse seiner Befestigungsschenkel (11) schwenkbar gelagert als Entriegelungshebel ausgebildet ist.

3. Obertisch-Durchleuchtungsgerät nach Anspruch 1 oder 2, **dadurch gekennzeichnet, dass** die Übertragungseinrichtung ein Bowdenzug (13) ist.

## Claims

1. An above-table transillumination device with an X-ray radiator rotatable around the horizontal axis of the radiator stand with an anti-rotation lock with a manually-operated unlocking lever, that is positioned at the front edge of the radiation carrier (7) away from the radiation stand (4) and is connected to the lever (14) of the anti-rotation lock, **characterized in that**, the unlocking lever is integrated into an essentially L-or C-shaped handle (10) with a grip arm lying in a vertical plane for swiveling the radiator carrier (7) .

2. An above-table transillumination device in accordance with Claim 1, **characterized in that** the handle (1) of the radiator carrier (7) is supported so that it can pivot around the axis of its attachment leg (11) and is designed as an unlocking lever.

3. An above-table transillumination device in accordance with Claim 1 or 2, **characterized in that** the transmission device is a Bowden cable (13)

## Revendications

1. Appareil de radiographie monté au-dessus d'une table et comprenant une source de rayonnement X qui peut tourner autour de l'axe horizontal du montant porteur de la source de rayonnement, et comporte un blocage de pivotement avec un levier de déblocage qui peut être actionné manuellement, qui est disposé sur le côté avant du support (7) de la source de rayonnement, éloigné du montant porteur (4) de la source de rayonnement, et qui est relié par l'intermédiaire d'un système de transmission, au levier (14) du blocage de pivotement,
**caractérisé en ce que** le levier de déblocage est intégré dans une poignée (10) sensiblement en forme de L ou de C avec une branche de préhension située dans un plan vertical, pour faire pivoter le support (7) de la source de rayonnement.

2. Appareil de radiographie monté au-dessus d'une table, selon la revendication 1, **caractérisé en ce que** la poignée (10) du support (7) de la source de rayonnement, est réalisée en tant que levier de déblocage en étant montée pivotante autour de l'axe de ses branches de fixation (11).

3. Appareil de radiographie monté au-dessus d'une table, selon la revendication 1 ou 2, **caractérisé en ce que** le système de transmission est un système de commande par câble sous gaine dit système Bowden.
